# FASCICULE DE BREVET EUROPEEN

(11) **EP 1 434 623 B1**
(45) Date de publication et mention de la délivrance du brevet: **26.03.2008**
(21) Numéro de dépôt: 02795304.1
(22) Date de dépôt: 11.10.2002
(51) Int. Cl.: A61N 1/30

(54) **DISPOSITIF DE DELIVRANCE DE MEDICAMENTS PAR IONTOPHORESE OU ELECTROPORATION INTRAOCULAIRE**
VORRICHTUNG ZUR VERABREICHUNG VON MEDIKAMENTEN DURCH INTRAOKULARE IONTOPHORESE ODER ELECTROPORATION
DEVICE FOR MEDICINE DELIVERY BY INTRAOCULAR IONTOPHORESIS OR ELECTROPORATION

(30) Priorité: 12.10.2001 FR 0113177
(43) Date de publication de la demande: 07.07.2004
(73) Titulaire: Eyegate Pharma SAS, 75012 Paris (FR); Institut National de la Santé et de la Recherche Médicale (INSERM), 75654 Paris Cedex 13 (FR)
(72) Inventeur: BEHAR, Francine, F-75016 Paris (FR); ROY, Pierre, F-75019 Paris (FR)
(74) Mandataire: Callon de Lamarck, Jean-Robert
(86) Numéro de dépôt international: PCT/FR2002/003473
(87) Numéro de publication internationale: WO 2003/043689

(56) Documents cités:
- EP-A- 1 127 586
- US-A- 4 564 016
- US-A- 6 001 088
- US-A- 6 101 411
- US-A- 6 154 671

## Description

L'invention concerne un dispositif d'applications d'un principe actif par iontophorèse ou électroporation destiné à la thérapie de l'oeil pour améliorer la délivrance intraoculaire de principes actifs en ophtalmologie.

La iontophorèse, comme l'électroporation, utilise le courant électrique pour permettre la diffusion d'une molécule chargée à travers une membrane biologique. Sous l'effet du courant électrique, la perméabilité de la membrane biologique est augmentée, ce qui permet le passage de molécules plus importantes, et le champ électrique pousse les molécules à travers cette membrane.

Actuellement, les dispositifs de iontophorèse oculaire existant sont des dispositifs périoculaires. Le document US-A-4 564 016 présente un tel dispositif comportant un ballonnet monté sur l'extrémité distale d'une sonde. Ce ballonnet permet de dégager l'espace rétrobulbaire (derrière l'oeil). Un tel dispositif présente l'inconvénient majeur de mettre sous pression l'oeil dont la pression normale est de 18 mmHg. Au-delà de 21 mmHg, les risques de glaucome aigu dû à une augmentation brusque de la pression oculaire sont importants, ce glaucome menant à la perte de la vision par endommagement du nerf optique.

D'autre part, ce type de dispositif, lors de son utilisation, engendre une pression dans les espaces périoculaires (rétro et péribulbaire). Cette pression peut entraîner une mauvaise irrigation sanguine par compression au niveau de la tête du nerf optique. Dans certains cas, cela peut aller jusqu'à une occlusion veineuse ou artérielle conduisant à une perte visuelle partielle ou totale.

Un autre inconvénient dû à un tel dispositif est que la paroi du globe oculaire à cet endroit est épaisse. De plus, ce dispositif ne permet pas de cibler avec précision les cellules ou les organes de l'oeil et la surface traitée est importante. Cela ne permet pas d'assurer un traitement correct et optimal d'une cible intraoculaire à traiter comme par exemple des cellules de la rétine.

Un but de l'invention est de fournir un dispositif d'application d'un principe actif par iontophorèse ou électroporation intraoculaire permettant de cibler avec précision la zone à traiter tout en évitant les risques de glaucome.

Pour cela, on prévoit, selon l'invention, un dispositif selon la revendication 1.

Ainsi, la présence de moyens d'injection coordonnés à des moyens d'aspiration permet de conserver :
- une pression constante et définie au sein du réservoir,
- un volume constant et défini si le réservoir est apte à se déformer.

Ceci est compatible avec une introduction du réservoir au sein du globe oculaire sans augmentation sensible de la pression du globe. Ceci évite les risques de glaucome et permet d'approcher avec précision les cellules cibles à traiter et de ne traiter qu'icelles, sans qu'il y est de diffusion de principe actif dans tout l'espace oculaire.

Avantageusement, le dispositif présente au moins une des caractéristiques suivantes :
- le dispositif comporte un tube d'injection et un tube d'aspiration s'étendant l'un dans l'autre et aptes à être raccordés au réservoir,
- les moyens de diffusion sont disposés à une extrémité distale d'une sonde,
- le réservoir est disposé à l'extrémité distale,
- le dispositif comporte un tube d'injection et un tube d'aspiration s'étendant à l'intérieur d'une sonde,
- l'extrémité distale de la sonde forme un angle par rapport à la direction selon laquelle la sonde s'étend principalement,
- l'angle est compris entre 90° et 170°, permettant ainsi un contact avec la rétine tout en gardant une visibilité optimale de la manipulation à travers le cristallin,
- l'angle est de l'ordre de 135°,
- une électrode de iontophorèse ou d'électroporation s'étend dans une sonde, notamment à l'intérieur du réservoir.
- les moyens de diffusion comportent une paroi poreuse apte à laisser passer le principe actif, en particulier sous l'effet d'un courant de iontophorèse ou d'électroporation,
- le réservoir présente une paroi comportant au moins un orifice de diffusion,
- l'orifice est recouvert par une membrane perméable ou semi-perméable apte à laisser passer le principe actif, en particulier sous l'effet d'un courant de iontophorèse ou d'électroporation,
- l'orifice latéral est bouché du côté du réservoir par un bouchon en matériau absorbant apte à laisser passer le principe actif, en particulier sous l'effet d'un courant de iontophorèse ou d'électroporation,
- le dispositif comporte en outre une fibre optique apte à être reliée à une source lumineuse et agencée de sorte à éclairer l'environnement des moyens de diffusion, notamment les cellules cibles à traiter,
- le dispositif comprend une seconde fibre optique apte à être reliée à une caméra agencée de sorte à enregistrer des images de l'environnement des moyens de diffusion, notamment les cellules cibles à traiter.

On prévoit aussi, selon l'invention, une sonde pour l'application oculaire d'un principe actif par iontophorèse ou électroporation intraoculaire comportant un réservoir apte à recevoir une solution comprenant le principe actif, des moyens de diffusion du principe actif connectés au réservoir, la sonde comportant en outre un tube d'injection de la solution dans le réservoir et un tube d'aspiration d'un contenu du réservoir.

On prévoit en outre, selon l'invention, une méthode chirurgicale apte à mettre en oeuvre le dispositif présentant au moins l'une des caractéristiques précitées et présentant au moins une des étapes suivantes :
- mise en place d'une électrode de retour reliée à un générateur sur les tissus avoisinant le globe oculaire à traiter,
- l'électrode de retour est une électrode de type cutanée,
- l'électrode de retour est apte à être positionnée sur tout ou partie de la surface externe du globe oculaire,
- incision de la sclère du globe oculaire à traiter,
- introduction par l'incision de la sonde dans le vitré,
- positionnement de l'extrémité distale de la sonde dans le voisinage de la zone à traiter du globe oculaire,
- injection dans le réservoir d'une solution contenant le principe actif par les moyens d'injection et régulation de la pression et/ou du volume du réservoir avec les moyens d'aspiration,
- mise sous tension de l'électrode de la sonde reliée au générateur durant un temps donnée et pour une tension donnée,
- arrêt du générateur,
- retrait de la sonde, ainsi que de l'électrode de retour,
- fermeture de l'incision de la sclère.

D'autres caractéristiques et avantages de l'invention apparaîtront lors de la description ci-après d'un mode préféré de réalisation ainsi que de variantes. Aux dessins annexés :
- la figure 1 est une représentation schématique d'un dispositif d'application intraoculaire selon l'invention,
- la figure 2a est une vue partielle de l'extrémité distale d'une sonde selon un premier mode de réalisation de l'invention,
- la figure 2b est une coupe selon IIb-IIb de l'extrémité distale de la sonde de la figure 2a,
- la figure 3a est une vue partielle de l'extrémité distale de la sonde selon un deuxième mode de réalisation de l'invention,
- la figure 3b est une vue en coupe selon IIIb-IIIb de l'extrémité distale de la sonde de la figure 3a,
- la figure 4 est une vue en coupe de l'extrémité distale d'une sonde selon un troisième mode de réalisation de l'invention,
- les figures 5a à 5e sont différents modes de réalisation de l'extrémité distale et du réservoir d'une sonde selon l'invention,
- la figure 6 représente une sonde selon une variante de réalisation de l'invention,
- la figure 7 est une coupe anatomique d'un globe oculaire, et
- la figure 8 est une coupe anatomique du globe oculaire montrant l'insertion lors de l'utilisation d'une sonde selon l'invention.

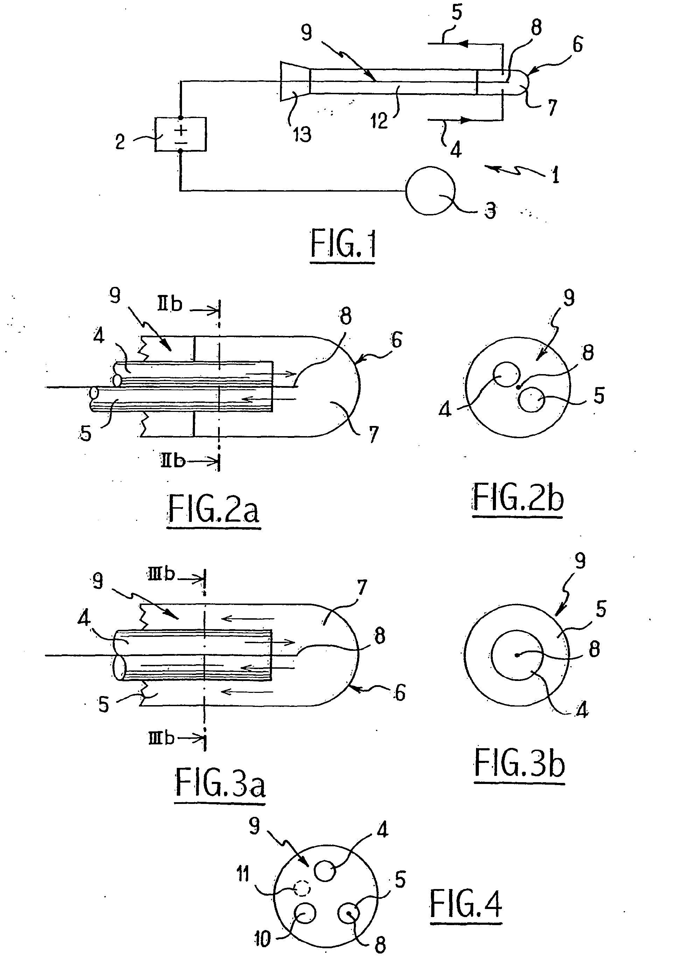

En référence à la figure 1, nous allons décrire un dispositif d'application oculaire d'un principe actif par iontophorèse ou électroporation intraoculaire selon l'invention. Le dispositif 1 comporte un générateur de courant 2 relié à une première électrode 3, dite électrode de retour, et à une deuxième électrode 8, dite électrode principale ou active. A cette électrode principale 8 est associée un réservoir 7 qui est apte à recevoir le principe actif à appliquer ou bien une solution contenant le principe actif. Le réservoir 7 comporte des moyens d'injection 4 ainsi que des moyens d'aspiration 5. D'autre part, le réservoir comporte une paroi poreuse 6 apte à ne laisser passer le principe actif que lors de la iontophorèse ou de l'électroporation. Le réservoir 7 peut être monté sur une sonde 9. De préférence, le réservoir se trouve au niveau de l'extrémité distale de la sonde 9.

L'électrode de retour 3 est destinée à fermer le circuit électrique formé par le générateur, l'électrode principale et les tissus organiques. L'électrode 3 est placée en regard de l'électrode principale 8 et des tissus organiques à traiter ou bien préférentiellement dans l'environnement proche de ces tissus à traiter, comme nous le verrons ultérieurement. Dans ce cas, l'électrode de retour 3 est une électrode cutanée de type TENS (Transcutaneous Electrical Nerve Stimulation ou stimulation nerveuse électrique transcutanée). Elle est composée principalement d'un adhésif conducteur cutané et d'un film conducteur en carbone relié au générateur par un cordon électrique.

L'électrode principale 8 est constituée de manière préférentielle d'un matériau choisi parmi une large variété de matériaux conducteurs électriquement. Ces matériaux peuvent être:
- inertes. Ils ne se corrodent pas de façon électrochimique du fait de la présence du courant électrique lors du fonctionnement du dispositif. Ces matériaux inertes sont de l'acier inoxydable, du platine, de l'or, du carbone, du tungstène, etc...
- sacrificiels. Ils se transforment, sous l'action du courant électrique lors du fonctionnement du dispositif, en ions métalliques qui se précipitent, évitant l'électrolyse de la solution comprenant le principe actif. Ces matériaux sont l'argent, le cuivre, etc...
Selon d'autres variantes de réalisations, l'électrode principale 8 est constituée d'encre ou de polymère conducteur, ou encore de particules conductrices dispersées dans une matrice.
D'autre part, l'électrode principale 8 se présente sous la forme soit d'un fil, soit d'un film, soit d'une plaque, soit encore d'un matériau tressé.

Le générateur de courant 2 délivre un courant continu présentant une intensité comprise entre 0.5 et 5mA environs, pendant une période allant de 0.5 à 10 minutes environ. En fonction de la résistance des tissus organiques participant au circuit, résistance susceptible de varier durant la iontophorèse, la tension délivrée par le générateur 2 s'adapte selon la loi d'Ohm U=R.I, où U est la tension en Volts, R la résistance en Ohms et I l'intensité en Ampères ; cependant la tension délivrée par le générateur 2 ne peut jamais excéder 20V.

Selon une variante de réalisation, le générateur 2 peut être un générateur de courant alternatif. Le courant alternatif a pour avantage d'éviter une variation du pH due à des phénomènes d'oxydo-réduction au niveau de l'électrode principale. La plage de fréquence de ce courant alternatif est choisie de manière à permettre une perméabilité optimale des tissus à traiter. Dans ce cas, l'électrode de retour 3 est de préférence une électrode de type ECG (ElectroCardioGramme) et composée d'un adhésif cutané et d'un film en Ag/AgCl présentant une impédance très faible.

Selon une autre variante de réalisation, le générateur 2 peut fournir un profil de courant présentant des pics de tension très élevée, compris entre 50 et 2500V environs, et de très courte durée, comprise entre 0.01 et 0.1s environs, et ce, sous faible intensité. Ce type de profil est couramment utilisé lors de l'électroporation.

La sonde 9 comporte, ici, trois parties principales : une embase de connexion 13, un tube 12 et une extrémité distale 7 comportant le réservoir 7. L'embase de connexion 13 est de typé Luer femelle qui est le standard universel de connexion des cathéters intraveineuses. Cette embase est de forme générale de cône de diamètre d'entrée de l'ordre de 4 mm et de conicité de l'ordre de 6%. Le tube de la sonde est réalisé en matériau polymère biocompatible de préférence comme du PolyChlorure de Vinyle, du Polyéthylène, du PolyPropylène, du PolyAmide, du PolyEther Bloc Amide, du Polyuréthanne ou du Silicone, suivant les caractéristiques de dureté et de transparence souhaitées.

En référence aux figures 2a et 2b, nous allons décrire un premier mode de réalisation de la sonde 9. La sonde 9 comprend une tubulure d'injection 4 débouchant dans le réservoir 7 situé à l'extrémité distale de la sonde 9. Cette tubulure 4 chemine le long de la sonde 9 sensiblement de manière parallèle à un axe longitudinal de ladite sonde. De même, une tubulure d'aspiration 5 chemine de manière sensiblement parallèle à la tubulure 4 au sein de la sonde 9 et plonge au sein du réservoir 7. Comme illustré à la figure 2b, préférentiellement la sonde est de section circulaire ainsi que les tubulures 4 et 5. Le diamètre de cette section circulaire est compris entre environ 0.9mm (Gauge 20) et environ 2.1mm (Gauge 14). La longueur de la sonde 9 est comprise entre 20 et 50 mm environ.

En référence aux figures 3a et 3b, nous allons décrire un second mode de réalisation de la sonde 9. La sonde 9, comme précédemment, présente une tubulure d'injection 4 débouchant dans le réservoir 7 situé à l'extrémité distale de la sonde. La tubulure 4 présente un axe longitudinal qui se confond quasiment avec un axe longitudinal de la sonde 9. Ce dernier fait office de tubulure d'aspiration 5. Sensiblement le long de l'axe longitudinal de la tubulure 4 et de la sonde 9 se trouve l'électrode 8. De préférence, la sonde 9 ainsi que la tubulure 4 présentent une section circulaire. Ainsi l'électrode 8, la tubulure d'injection 4 et la sonde 9 sont sensiblement coaxiaux.

En référence à la figure 4, nous allons décrire un troisième mode de réalisation de la sonde 9. Comme illustré sur cette figure, la sonde 9 présente une section circulaire. Au sein de cette sonde 9, cheminent de manière sensiblement parallèle à un axe longitudinal de la sonde 9 une tubulure d'injection 4 de section préférentiellement circulaire, une tubulure d'aspiration 5 de section préférentiellement circulaire dans laquelle se trouve l'électrode 8. De plus, la sonde 9 présente une première fibre optique 10 qui est reliée à une source lumineuse non représentée de manière à acheminer des rayons lumineux au voisinage de l'extrémité distale de la sonde 9 pour éclairer l'environnement de la cible à traiter. Enfin, la sonde 9 peut présenter une seconde fibre optique 11 reliée à une caméra et apte à acheminer vers cette caméra des rayons lumineux provenant de l'extrémité distale de la sonde de manière à faire enregistrer par la caméra des images de l'environnement de la cible à traiter. La présence de ces fibres optiques permet d'améliorer la précision avec laquelle sera manipulée la sonde pour traiter la cible par iontophorèse ou électroporation.

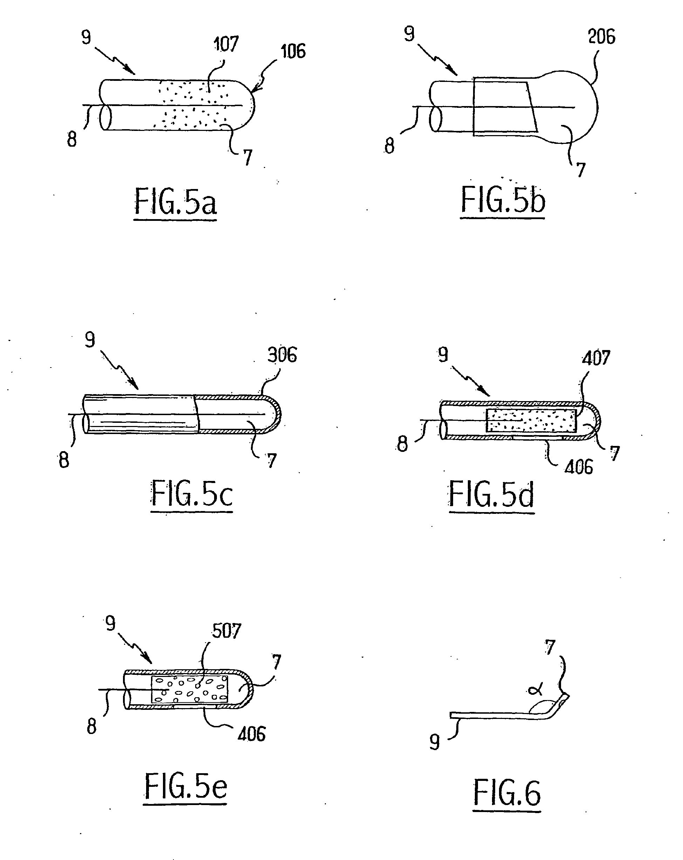

En référence aux figures 5a à 5e, nous allons décrire différents modes de réalisation de la paroi du réservoir.

En référence à la figure 5a, la paroi 106 du réservoir 7 présente une multitude de micro orifices 107 traversant la paroi 106. Ces micro-orifices 107 sont aptes à laisser passer les molécules du principe actif sous l'action du champ électrique émis par l'électrode 8 qui permet d'effectuer l'iontophorèse ou l'électroporation. De préférence, les différents micro-orifices 107 sont uniformément répartis sur une bande entourant le réservoir dont la largeur ne dépasse pas la taille du réservoir. De préférence encore, différents micro-orifices 107 sont situés sur une portion limitée de la paroi du réservoir. Les micro-orifices présentent un diamètre moyen compris entre 0.01 et 0.1 mm environ.

En référence à la figure 5b, la sonde 9 est ouvert à son extrémité distale 9, un ballon 206 est enfilé sur l'extrémité distale ouverte de la sonde 9, le ballon faisant office de réservoir 7. La membrane formant le ballon 206 est une membrane de préférence semi-perméable ou perméable ou encore micro poreuse apte à ne laisser passer les molécules du principe actif que sous l'action du champ électrique généré par l'électrode 8 lors de la iontophorèse ou de l'électroporation.

Sur la figure 5c, l'extrémité distale de la sonde 9 présente un embout poreux 306 entourant le réservoir 7. L'embout poreux est apte à ne laisser passer les molécules du principe actif que sous l'effet du champ électrique généré par l'électrode 8 lors de la iontophorèse ou de l'électroporation.

Sur la figure 5d, l'extrémité distale de la sonde 9 présente un orifice débouchant 406 apte à faire communiquer le réservoir 7 avec l'extérieur de la sonde. De préférence, l'orifice 406 est situé sur une paroi latérale entourant le réservoir 7. Cet orifice 406 est bouché par une membrane de préférence perméable ou semi-perméable ou encore micro poreuse 407. Cette membrane 407 peut être située sur l'orifice 406 à l'intérieur du réservoir 7 ou bien positionnée sur l'orifice 406 à l'extérieur du réservoir 7. Préférentiellement dans ce cas la membrane pourra être un manchon enfilé sur l'extrémité distale de la sonde et venant recouvrir l'orifice 406. L'orifice traversant est réalisé par un perçage présentant un diamètre moyen compris entre 0.5 à 1mm environ. La membrane présente une porosité comprise entre 1 et 10 µm environ.

Sur la figure 5e, l'extrémité de la sonde 9 présente la même configuration d'extrémité que celle représentée sur la figure 5d. L'orifice 406 est ici bouché par un matériau absorbant 507 qui occupe la plus grande partie du réservoir 7. Le matériau absorbant 507, comme la membrane 407, est apte à ne laisser passer les molécules du principe actif que sous l'effet d'un champ électrique issu de l'électrode 8 lors de la iontophorèse ou de l'électroporation. Le matériau absorbant est de préférence de la mousse ou de l'éponge.

Dans tous les modes de réalisation de la sonde 9 précédemment décrits, l'électrode principale 8 doit être placée en regard soit de la membrane, soit des orifices de manière à permettre un écoulement optimal du courant électrique vers l'extérieur de la sonde 9, lors du fonctionnement.

D'autre part, selon les modes de réalisation ci-dessus décrits, la surface de traitement effective de cellules cibles est comprise entre environ 50µm de diamètre et environ 2mm de diamètre.

En référence à la figure 6, la sonde peut être coudée au niveau de son extrémité distale. L'angle α que forme l'extrémité distale avec le tube 12 de la sonde peut être compris entre 90°et 170°. De manière préférentielle, cet angle est sensiblement égal à 135°. Le choix de cet angle de 135° est fonction de la voie utilisée pour introduire la sonde au sein du globe oculaire, comme nous le verrons ci-dessous. L'angle est choisi de manière à ce que l'axe longitudinal de l'extrémité distale de la sonde 9 soit sensiblement parallèle à la surface que forment les cellules cibles à traiter. Dans une variante de réalisation non représentée, l'angle que forme l'extrémité distale de la sonde avec le tube de la sonde est choisi par l'opérateur au cours de la procédure chirurgicale, de manière à parfaire l'alignement précédent. Pour cela, l'extrémité distale de la sonde 9 comportant le réservoir 7 est monté à rotation autour d'un axe (non représenté), perpendiculaire à l'axe selon lequel s'étend principalement la sonde, sur le tube de la sonde 9, et le dispositif d'application comporte des moyens de mise en oeuvre de cette extrémité situés à l'extrémité proximale de ladite sonde.

Nous allons maintenant décrire l'utilisation pratique du dispositif d'application par iontophorèse ou électroporation intraoculaire selon l'invention.

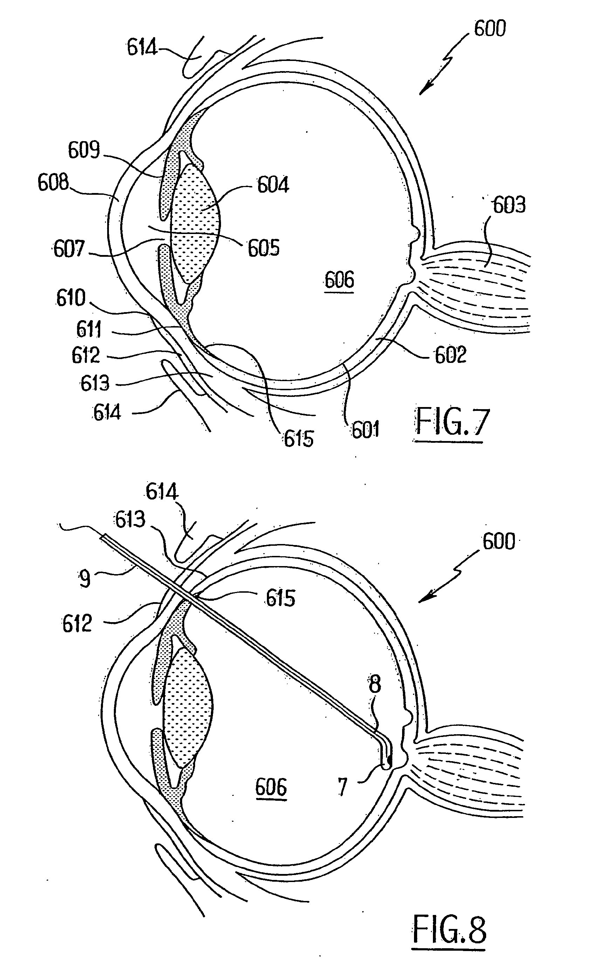

En référence à la figure 7, l'oeil 600 a la forme générale d'un ballon. La partie antérieure de la paroi est constituée d'une cornée transparente 608 derrière laquelle se trouve une pupille 607. Cette dernière est séparée de la cornée 608 par une chambre antérieure 605 comportant une humeur aqueuse. La pupille est fermé par un cristallin 604 transparent et conformé à la manière d'une lentille convergente. Le volume 606 situé derrière le cristallin 604 est appelé vitré. La paroi postérieure de l'oeil est constituée d'une première couche 601 formant la rétine fonctionnelle puis d'une seconde couche 602 appelée choroïde puis enfin d'une troisième couche 613 appelée sclère. A l'extrémité arrière du globe oculaire part un nerf optique 603. Le cristallin 604 est maintenu à l'avant par un iris 609 et est relié à la paroi du globe oculaire au niveau de la limite entre la cornée et la sclère par un corps ciliaire 611. Entre le point d'attache du corps ciliaire 611 et le début de la rétine fonctionnelle 601 se trouve une pars plana non fonctionnelle 615. A partir d' un limbe 610 entourant la cornée 608 se trouve une couche de tissu 612 appelée conjonctive s'étendant au-dessus de la sclère juste au niveau de l'implantation des paupières 614.

En référence à la figure 8, nous allons décrire le mode opératoire. Le chirurgien effectue une voie d'abord transclérale en effectuant une incision de la sclère au niveau de la *pars plana* non fonctionnelle 615. En effet, à cet endroit particulier, la paroi formant le globe oculaire est la moins épaisse. Ensuite, le chirurgien positionne l'électrode 3 du dispositif d'application 1 sur la peau du visage le plus proche possible de l'oeil, de préférence sur le front, la joue ou la paupière (il est aussi possible de positionner l'électrode de retour sous la conjonctive, au contact direct du globe oculaire, ou encore directement dans l'oeil). Ensuite il introduit, par l'incision effectuée dans la sclère, la sonde 9 qui pénètre alors dans le vitré 606. Le chirurgien a la possibilité de suivre l'introduction de la sonde soit directement à travers la cornée et le cristallin qui sont transparents ou bien à l'aide d'une caméra si la sonde est équipée de fibre optique à cet effet, ou bien encore à l'aide d'une lampe à fentes ou d'une lentille. L'introduction de la sonde 9 est réalisée de manière à ce que l'extrémité distale 7 courbée vienne au voisinage des cellules à traiter. Une fois la sonde 9 mis en place, le chirurgien fait circuler un courant dans l'électrode 8 pour effectuer la iontophorèse ou l'électroporation lors de laquelle une certaine quantité de principe actif, fonction de l'intensité du courant d'une part et du temps de mise sous tension, est transférée du réservoir 7 dans les cellules cibles à traiter. Ensuite, le chirurgien retire la sonde 9 puis referme son incision.

Comme nous avons pu le voir, la configuration de la sonde 9 ainsi que la voie d'abord utilisée permet d'appliquer le principe actif de façon très localisée, sans affecter les tissus qui ne sont pas à traiter.

L'indication principale pour l'utilisation du dispositif d'application 1 est l'occlusion de vaisseaux rétiniens, cause classique de la perte totale ou partielle de la vision, en particulier chez les personnes âgées. C'est aussi une des complications des rétinopathies diabétiques.

L'occlusion de vaisseaux rétiniens se présente sous deux formes :
- la forme ischémique, la plus rare (10% à 15% des cas), se manifeste par une baisse brutale d'acuité visuelle évoluant vers la néovascularisation et le glaucome,
- la forme oedémateuse, la plus fréquente (60% à 80% des cas), se manifeste par un brouillard visuel, et évolue soit vers une rémission dans le cas de patients jeunes, soit vers une forme chronique avec dégradation lente de la rétine, soit enfin vers la forme ischémique décrite précédemment.

L"occlusion de vaisseaux est considérée comme une urgence et se traite actuellement par des traitements fibrinolytiques (thrombolyse ou dissolution des caillots) par voie générale ou locale, ou par des traitements rhéologiques (soustraction d'une certaine quantité de sang) par voie générale, ou encore par des traitements par phocoagulation laser qui permettent de prévenir ou de faire régresser la néovascularisation d'une part et de lutter contre l'oedème maculaire d'autre part. Le cas de l'injection de fibrinolytiques par voie générale présente des complications hémorragiques importantes. Dans le cas d'injection de ces mêmes fibrinolytiques par voie locale (c'est-à-dire par voie intravitréenne), on observe la survenue d'hémorragies intraoculaires. Ainsi, le dispositif d'application par iontophorèse ou électroporation 1 selon l'invention permet l'injection de ces même fibrinolytiques sur le seul tissu cible à traiter tout en évitant les complications hémorragiques précédemment citées.

D'autres pathologies oculaires peuvent être traitées par un dispositif d'application par iontophorèse ou électroporation selon l'invention. Il s'agit des maladies dégénératives de la rétine liées à l'âge ainsi que des rétinopathies diabétiques en général. De nombreuses molécules sont en cours de développement pour freiner voir arrêter la néovascularisation observée lors de ces pathologies. Ces molécules peuvent être injectées en utilisant un dispositif selon l'invention. Cela permet d'augmenter les concentrations locales des ces médicaments d'une part et d'autre part de pouvoir passer des molécules de taille plus importante dans les tissus cibles, comme par exemple lors d'une administration localisée d'antimitotiques ou d'antiangiogéniques.

De même, le dispositif selon l'invention peut être utilisé pour la transfection par iontophorèse ou électroporation de plasmides ou de médicaments de thérapie génique (comme par exemple des oligonucléotides chimères ou antisens, ou encore des ribozymes) dont le mode d'action principal est la correction de gènes ou de fragments de gènes à l'intérieur des cellules cibles.

Bien entendu, on pourra apporter à l'invention de nombreuses modifications sans pour autant sortir du cadre de celle-ci.

## Revendications

1. Dispositif d'application oculaire d'un principe actif par iontophorèse ou électroporation intraoculaire comprenant :
- un réservoir (7) apte à recevoir une solution comprenant le principe actif,
- des moyens de diffusion (8,6; 106; 206; 306; 406, 307;507) du principe actif connectés au réservoir,
- des moyens d'injection (4) de la solution dans le réservoir, ainsi que
- des moyens (5) aptes à exercer une aspiration d'un contenu du réservoir pendant une injection de la solution dans celui-ci par les moyens d'injection,
**caractérisé en ce que** le réservoir est agencé pour être au moins en partie introduit dans un globe oculaire.

2. Dispositif selon la revendication 1, **caractérisé en ce qu'**il comporte un tube d'injection (4) et un tube d'aspiration (5) s'étendant l'un dans l'autre et aptes à être raccordés au réservoir.

3. Dispositif selon l'une des revendications 1 à 2, **caractérisé en ce que** les moyens de diffusion comportent une paroi poreuse (6;106;306) apte à laisser passer le principe actif, en particulier sous l'effet d'un courant de iontophorèse ou d'électroporation.

4. Dispositif selon l'une des revendications 1 à 2, **caractérisé en ce que** le réservoir présente une paroi comportant au moins un orifice de diffusion (107;406).

5. Dispositif selon la revendication 4, **caractérisé en ce que** l'orifice (406) est recouvert par une membrane perméable ou semi-perméable (407) apte à laisser passer le principe actif, en particulier sous l'effet d'un courant de iontophorèse ou d'électroporation.

6. Dispositif selon la revendication 4 ou 5, **caractérisé en ce que** l'orifice latéral est bouché du coté du réservoir par un bouchon (507) en matériau absorbant apte à laisser passer le principe actif, en particulier sous l'effet d'un courant de iontophorèse ou d'électroporation.

7. Dispositif selon l'une des revendications 1 à 6 **caractérisé en ce qu'**il comporte en outre une fibre optique (10) apte à être reliée à une source lumineuse et agencée de sorte à éclairer l'environnement des moyens de diffusion, notamment des cellules cibles à traiter.

8. Dispositif selon la revendication 7, **caractérisé en ce qu'**il comprend une deuxième fibre optique (11) apte à être reliée à une caméra et agencée de sorte à enregistrer des images de l'environnement des moyens de diffusion, notamment des cellules cibles à traiter.

9. Dispositif selon l'une des revendications précédentes, **caractérisé en ce que** le dispositif est une sonde pour l'application oculaire d'un principe actif par iontophorèse ou électroporation intraoculaire, que les moyens d'injection comportent un tube d'injection et les moyens d'aspiration comportent un tube d'aspiration.

10. Dispositif selon la revendication précédente, **caractérisé en ce que** les moyens de diffusion sont disposés à une extrémité distale (7) de la sonde (9).

11. Dispositif selon la revendication précédente, **caractérisé en ce que** le réservoir (7) est disposé à l'extrémité distale.

12. Dispositif selon l'une des revendications 9-11, **caractérisé en ce qu'**une électrode de iontophorèse ou d'électroporation (8) s'étend dans la sonde (9).

13. Dispositif selon la revendication 10, **caractérisé en ce que** l'extrémité distale de la sonde forme un angle (α) par rapport à la direction selon laquelle la sonde s'étend principalement.

14. Dispositif selon la revendication 13, **caractérisé en ce que** l'angle est compris entre 90° et 170°.

15. Dispositif selon la revendication 14, **caractérisé en ce que** l'angle est de l'ordre de 135°.

## Claims

1. A device for ocular application of an active ingredient by intraocular iontophoresis or electroporation comprising:
- a reservoir (7) capable of receiving a solution comprising the active ingredient,
- means (8, 6; 106; 206; 306; 406, 307; 507) for diffusing the active ingredient, connected to the reservoir,
- means (4) for injecting the solution into the reservoir, as well as
- means (5) capable of exerting suction of a content of the reservoir during injection of the solution into the latter by the injection means,
**characterized in that** the reservoir is laid out so as to be at least partly introduced into an eye globe.

2. The device according to claim 1, **characterized in that** it includes an injection tube (4) and a suction tube (5) extending into each other and capable of being connected to the reservoir.

3. The device according to any of claims 1 to 2, **characterized in that** the diffusion means include a porous wall (6; 106; 306) capable of letting through the active ingredient, in particular under the effect of an iontophoresis or electroporation current.

4. The device according to any of claims 1 to 2, **characterized in that** the reservoir has a wall including at least a diffusion orifice (107; 406).

5. The device according to claim 4, **characterized in that** the orifice (406) is covered by a permeable or semi-permeable membrane (407) capable of letting through the active ingredient, in particular under the effect of an iontophoresis or electroporation current.

6. The device according to claim 4 or 5, **characterized in that** the side orifice is blocked on the reservoir side by a plug (507) in an absorbing material capable of letting through the active ingredient, in particular under the effect of iontophoresis or electroporation current.

7. The device according to any of claims 1 to 6, **characterized in that** it further comprises an optical fiber (10) capable of being connected to a light source and laid out so as to illuminate the environment of the diffusion means, notably of the target cells to be treated.

8. The device according to claim 7, **characterized in that** it comprises a second optical fiber (11) capable of being connected to a video camera and laid out so as to record images of the environment of the diffusion means, notably of the target cells to be treated.

9. The device according to any of the preceding claims, **characterized in that** the device is a probe for ocularly applying an active ingredient by intraocular iontophoresis or electroporation, **characterized in that** the injection means include an injection tube and the suction means include a suction tube.

10. The device according to the preceding claim, **characterized in that** the diffusion means are positioned at a distal end (7) of the probe (9).

11. The device according to the preceding claim, **characterized in that** the reservoir (7) is positioned at the distal end.

12. The device according to any of claims 9 to 11, **characterized in that** an iontophoresis or electroporation electrode (8) extends into the probe (9).

13. The device according to claim 10, **characterized in that** the distal end of the probe forms an angle the angle (α) relatively to the direction along which the probe mainly extends.

14. The device according to claim 13, **characterized in that** the angle is comprised between 90° and 170°.

15. The device according to claim 14, **characterized in that** the angle is of the order of 135°.

## Patentansprüche

1. Vorrichtung zur okularen Verabreichung eines Wirkstoffprinzips durch intraokulare Iontophorese oder Elektroporation, umfassend:
- einen Vorratsbehälter (7), der imstande ist, eine Lösung aufzunehmen, die das Wirkstoffprinzip umfasst,
- mit dem Vorratsbehälter verbundene Mittel (8, 6; 106; 206; 306; 406, 307; 507) zur Diffusion des Wirkstoffprinzips,
- Mittel zur Injektion (4) der Lösung im Vorratsbehälter sowie
- Mittel (5), die imstande sind, eine Ansaugung eines Inhalts des Vorratsbehälters während einer Injektion der Lösung in demselben durch die Injektionsmittel durchzuführen,
**dadurch gekennzeichnet, dass** der Vorratsbehälter aufgebaut ist, um mindestens teilweise in einen Augapfel eingeführt zu werden.

2. Vorrichtung nach Anspruch 1, **dadurch gekennzeichnet, dass** sie ein Injektionsrohr (4) und ein Ansaugrohr (5) umfasst, die sich ineinander erstrecken und imstande sind, mit dem Vorratsbehälter verbunden zu sein.

3. Vorrichtung nach einem der Ansprüche 1 bis 2, **dadurch gekennzeichnet, dass** die Diffusionsmittel eine poröse Wand (6; 106; 306) aufweisen, die imstande ist, das Wirkstoffprinzip passieren zu lassen, vor allem unter der Wirkung eines Iontophorese- oder Elektroporationsstroms.

4. Vorrichtung nach einem der Ansprüche 1 bis 2, **dadurch gekennzeichnet, dass** der Vorratsbehälter eine Wand aufweist, die mindestens eine Diffusionsöffnung (107; 406) aufweist.

5. Vorrichtung nach Anspruch 4, **dadurch gekennzeichnet, dass** die Öffnung (406) von einer durchlässigen oder halbdurchlässigen Membran (407) bedeckt ist, die imstande ist, das Wirkstoffprinzip passieren zu lassen, insbesondere unter Einwirkung eines Iontophorese- oder Elektroporationsstroms.

6. Vorrichtung nach Anspruch 4 oder 5, **dadurch gekennzeichnet, dass** die seitliche Öffnung auf der Seite des Vorratsbehälters durch einen Stopfen (507) aus einem absorbierenden Material verschlossen ist, der imstande ist, das Wirkstoffprinzip passieren zu lassen, insbesondere unter Einwirkung eines Iontophorese- oder Elektroporationsstroms.

7. Vorrichtung nach einem der Ansprüche 1 bis 6, **dadurch gekennzeichnet, dass** sie im weiteren eine optische Faser (10) aufweist, die imstande ist, mit einer Lichtquelle verbunden zu werden und die derart aufgebaut ist, um die Umgebung der Diffusionsmittel zu beleuchten, vor allem der zu behandelnden Zielzellen.

8. Vorrichtung nach Anspruch 7, **dadurch gekennzeichnet, dass** sie eine zweite optische Faser (11) aufweist, die imstande ist, mit einer Kamera verbunden zu werden und die derart aufgebaut ist, um Bilder von der Umgebung der Diffusionsmittel aufzuzeichnen, vor allem der zu behandelnden Zielzellen.

9. Vorrichtung nach einem der vorangehenden Ansprüche, **dadurch gekennzeichnet, dass** die Vorrichtung eine Sonde zur okularen Verabreichung eines Wirkstoffprinzips durch intraokulare Iontophorese oder Elektroporation ist, **dadurch gekennzeichnet, dass** die Injektionsmittel ein Injektionsrohr aufweisen und die Ansaugmittel ein Ansaugrohr aufweisen.

10. Vorrichtung nach dem vorangehendem Anspruch, **dadurch gekennzeichnet, dass** die Diffusionsmittel an einem distalen Ende (7) der Sonde (9) angeordnet sind.

11. Vorrichtung nach dem vorangehenden Anspruch, **dadurch gekennzeichnet, dass** der Vorratsbehälter (7) am distalen Ende angeordnet ist.

12. Vorrichtung nach einem der Ansprüche 9 bis 11, **dadurch gekennzeichnet, dass** sich eine Elektrode für die Iontophorese oder Elektroporation (8) in der Sonde (9) erstreckt.

13. Vorrichtung nach Anspruch 10, **dadurch gekennzeichnet, dass** das distale Ende der Sonde im Verhältnis zur Richtung, in die sich die Sonde hauptsächlich erstreckt, einen Winkel (α) bildet.

14. Vorrichtung nach Anspruch 13, **dadurch gekennzeichnet, dass** der Winkel zwischen 90° und 170° beträgt.

15. Vorrichtung nach Anspruch 14, **dadurch gekennzeichnet, dass** der Winkel in der Größenordnung von 135° ist.
